# EUROPEAN PATENT SPECIFICATION

(11) **EP 3 128 977 B1**
(45) Date of publication and mention of the grant of the patent: **31.01.2018**
(21) Application number: 15716676.0
(22) Date of filing: 01.04.2015
(51) Int. Cl.: A61F 13/511, A61F 13/514, A61F 13/533

(54) **EMBOSSING APPARATUS AND METHOD**
PRÄGEVORRICHTUNG UND VERFAHREN
APPAREIL ET PROCÉDÉ DE GAUFRAGE

(30) Priority: 08.04.2014 US 201414247631
(43) Date of publication of application: 15.02.2017
(73) Proprietor: The Procter & Gamble Company, Cincinnati, OH 45202 (US)
(72) Inventor: DENG, Rong, Cincinnati, Ohio 45202 (US); WILSON, Gregory, James, Cincinnati, Ohio 45202 (US); ISBURGH, Robert, Karl, Cincinnati, Ohio 45202 (US)
(74) Representative: Kremer, Véronique Marie Joséphine
(86) International application number: PCT/US2015/023854
(87) International publication number: WO 2015/157063

(56) References cited:
- EP-A1- 2 485 696
- EP-A1- 2 485 697
- US-A1- 2005 035 492
- US-A1- 2006 286 885

## Description

### FIELD OF THE INVENTION

The present disclosure relates to methods for manufacturing absorbent articles, and more particularly, to apparatuses and methods for embossing substrates that may be used as components of absorbent articles.

### BACKGROUND OF THE INVENTION

Along an assembly line, various types of articles, for example sanitary napkins, diapers, and other absorbent articles, may be assembled by adding components to and/or otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. In some cases, individual components created from advancing web or webs are combined with other individual components created from other advancing web or webs. Webs of material and component parts used to manufacture diapers may include: backsheets, topsheets, leg cuffs, waist bands, absorbent core components, front and/or back ears, fastening components, and various types of elastic webs and components such as leg elastics, barrier leg cuff elastics, stretch side panels, and waist elastics. Webs of material and component parts used to manufacture sanitary napkins may include: backsheets, topsheets, secondary topsheets, absorbent core components, release paper wrappers, and the like. Once the desired component parts are assembled, the advancing web(s) and component parts are subjected to a final knife cut to separate the web(s) into discrete articles.

During the assembly process, components, combinations of components, and/or advancing webs of material may be embossed. Rotary embossing systems have traditionally been used to emboss the webs that form absorbent articles. Typical embossing systems have included rotary embossing rolls and cooperating, rotary anvil rolls. Different embossment dies can be fixed to rotary embossing rolls to produce a variety of desired embossment patterns for absorbent articles. One style of embossing is deep channel embossing. Deep channel embossments in an absorbent article provide improved fit of the absorbent article to the wearer's body, which is important in delivering superior protection performance. Deep channel embossments also provide a pleasant feminine design to the wearer. Further, the embossed area article. In addition, the process of forming deep channel embossments can produce embossments not only in the surface contacted by the rotary embossing rolls (typically the body facing surface including the topsheet), but also in the opposing surface (typically the garment facing surface).

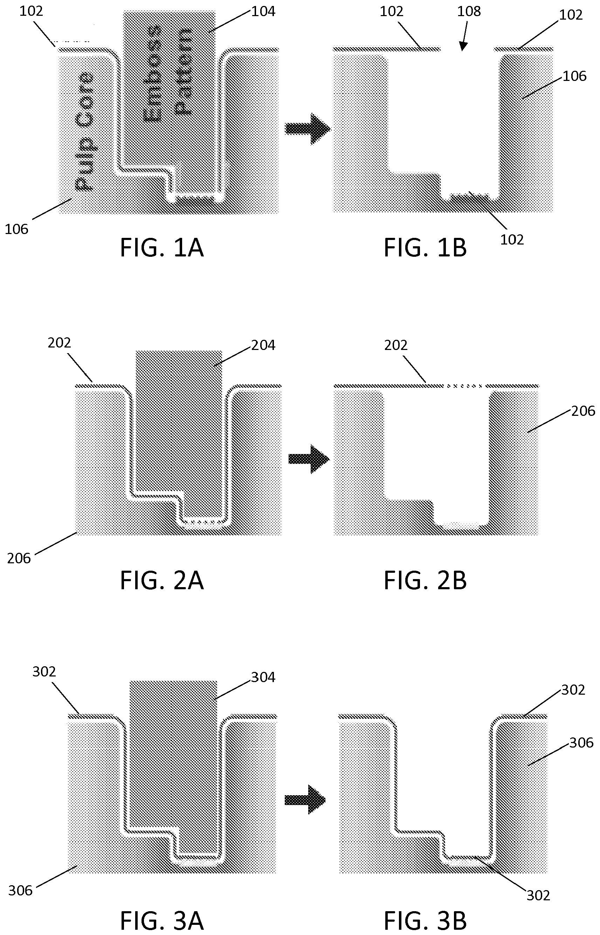

A typical embossed absorbent article, such as feminine sanitary napkin, comprises a topsheet, absorbent core, and backsheet; the backsheet is usually attached to the top sheet prior to the embossment of the topsheet and absorbent core. Embossing may damage the topsheet due to the embossing nubs forming holes and/or tears in or around the emboss sites. Embossing tooling may comprise sharp edges and may tear, cut, or weaken an embossed absorbent article in areas adjacent to the embossing, e.g., embossed channels. If the topsheet is torn by the embossing tooling, it may adversely affect the topsheet attachment to the core beneath it and the torn topsheet portion may pop out of the channel. This may cause the channel to lose its intended depth of perception. And, this often creates a consumer-noticeable defective product. For example, if a topsheet 102 gets too much strain from an embossing nub 104, it can tear or have a punched-thought effect (FIGS. 1A and 1B) wherein the topsheet does not stay embossed to the core 106 and just gives the illusion of being apertured or slit. In another example, if the embossing strength is not high enough between the topsheet 202 and the core 206, the topsheet may detach from the core due to topsheet tension (FIGS. 2A and 2B). Also, as the web basis weight of the laminate decreases, channels may become more susceptible to defects such as tearing and pinholes at relatively high nip pressures. This is an increasing problem as lower basis weight and/or lower cost web materials are used.

Consequently, it would be beneficial to provide a method and apparatus for embossing substrates that produces emboss sites, such as deep channels, with relatively low damage to the topsheet. There is a desire for embossing nubs which minimize the applied process strain on a substrate, so that this applied process strain is less than the failure strain of the substrate. There is a need to reduce or eliminate substrate tearing as a result of embossing. There is a desire for nubs that are customized for substrates to be bonded such that tears are minimized or eliminated.

EP 2 485 697 discloses an apparatus for embossing an absorbent article.

### SUMMARY OF THE INVENTION

The present disclosure relates to methods and apparatuses for embossing substrates. According to an aspect of the invention, there is provided an apparatus according to claim 1. In one embodiment, an apparatus for embossing substrates comprises: an anvil roll; and an embossing roll comprising a base surface and plurality of nubs extending from the base surface, each of the plurality of nubs comprising: a sidewall; an embossing surface having a width; and a shoulder connecting the sidewall to the embossing surface; wherein the shoulder comprises a radius of from 0.1mm to 1mm; wherein the embossing surface width is from 0.25mm to 4mm; and wherein the sidewall is substantially tapered from the base surface. In another aspect of the invention, there is provided a method according to claim 7. In an embodiment, the method for embossing substrates comprises the steps of: providing an anvil roll; providing an embossing roll comprising a base surface and plurality of nubs extending from the base surface, each of the plurality of nubs comprising: a sidewall; an embossing surface having a width; and a shoulder connecting the sidewall to the embossing surface; wherein the shoulder is comprises a radius of from 0.1mm to 1mm; wherein the embossing surface width is from 0.25mm to 4mm; and wherein the sidewall is substantially tapered from the base surface; and advancing a topsheet an absorbent core between the anvil roll and the embossing roll so that emboss regions are formed via the plurality of nubs.

### BRIEF DESCRIPTION OF THE DRAWINGS

The following detailed description of specific embodiments of the present invention can be best understood when read in conjunction with the drawings enclosed herewith.
FIG. 1A is an enlarged cross-section view of a first prior art embossing nub embossing a topsheet onto an absorbent core.
FIG. 1B shows the topsheet and absorbent core of FIG. 1A after the embossing process is complete.
FIG. 2A is an enlarged cross-section view of a second prior art embossing nub embossing a topsheet onto an absorbent core.
FIG. 2B shows the topsheet and absorbent core of FIG. 2A after the embossing process is complete.
FIG. 3A is enlarged cross-section view of a new embossing nub embossing a topsheet onto an absorbent core.
FIG. 3B shows the topsheet and absorbent core of FIG. 3A after the embossing process is complete.
FIG. 4 shows a schematic side view of a method for embossing an absorbent core.
FIG. 5 is a top view (including a partial cut-away) of the body-facing surface of a feminine sanitary napkin having an embossment region.
FIG. 6 is a cross-sectional view of the sanitary napkin of FIG. 5 along the line 6-6.
FIG. 7 is a side view of an exemplary rotary embossing device.
FIG. 8 is a top view of an embossing member.
FIG. 9 is a cross-sectional view of an embossing roll, showing an embossing nub.
FIG. 10 is an enlarged cross-sectional view of the embossing nub of FIG. 9.
FIG. 11 shows three exemplary obround embossing nubs.

### DETAILED DESCRIPTION OF THE INVENTION

The present disclosure relates to a method and apparatus for manufacturing absorbent articles, such as sanitary napkins or incontinence articles, and in particular, for embossing one or more substrates which make up the absorbent articles. This method and apparatus for embossing substrates produces embossment regions, or sites, such as deep channels, while imparting relatively low damage to the topsheet. A method for producing the embossed absorbent article includes moving a topsheet and an absorbent core having a body facing surface and a garment facing surface, in the machine direction, such that they are embossed by a rotary embossing device having one or more embossing nubs in operative contact with a rotary anvil. The embossing nubs contact the body facing surface of the topsheet, forming embossment regions in the body facing surface of the topsheet and absorbent core and forming depression regions corresponding to the embossment regions in the garment facing surface of the absorbent core. FIG. 3A is enlarged cross-section view of a new embossing nub 304 embossing a topsheet 302 onto an absorbent core 306. FIG. 3B shows the topsheet 302 and absorbent core 306 of FIG. 3A after the embossing process is complete. This is an exemplary embossing nub which minimizes the applied process strain on a substrate, so that the applied process strain is less than the failure strain of the substrate. This reduces, and often eliminates, substrate tearing as a result of embossing. These customized nubs are discussed further below.

As used herein, the term "absorbent article" includes disposable articles such as sanitary napkins, panty liners, tampons, interlabial devices, wound dressings, diapers, adult incontinence articles, wipes, and the like. At least some of such absorbent articles are intended for the absorption of body liquids, such as menses or blood, vaginal discharges, urine, and feces. Wipes may be used to absorb body liquids, or may be used for other purposes, such as for cleaning surfaces. Various absorbent articles described above will typically comprise a liquid pervious topsheet, a liquid impervious backsheet joined to the topsheet, and an absorbent core between the topsheet and backsheet.

Absorbent articles, and their individual components, such as a liquid pervious topsheet, a substantially liquid impervious backsheet joined to the topsheet, and an absorbent core positioned and held between the topsheet and the backsheet, have a body facing surface and a garment facing surface. As used herein, "body-facing surface" means that surface of the article or component which is intended to be disposed toward or placed adjacent to the body of the wearer during ordinary use, while the "garment facing surface" is on the opposite side, and is intended to be disposed to face away from the wearer's body during ordinary use. The garment facing surface may be arranged to face toward or placed adjacent to the wearer's undergarments when the absorbent article is worn. In general the topsheet is operatively permeable to the liquids that are intended to be held or stored by the absorbent article, and the backsheet may be substantially impermeable or otherwise operatively impermeable to the intended liquids. The absorbent article may also include other components, such as a secondary topsheet, liquid wicking layers, liquid distribution layers, barrier layers, and the like, as well as combinations thereof.

As used herein, the term "nub" refers to any element on the surface of a roll that is capable of embossing one or more substrates.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a web, layer or layers or fibrous materials, nonwovens, films and foils such as polymeric films or metallic foils. These materials may be used alone or may comprise two or more layers laminated together (a "composite substrate"). As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

### Embossing Process and Embossed Article

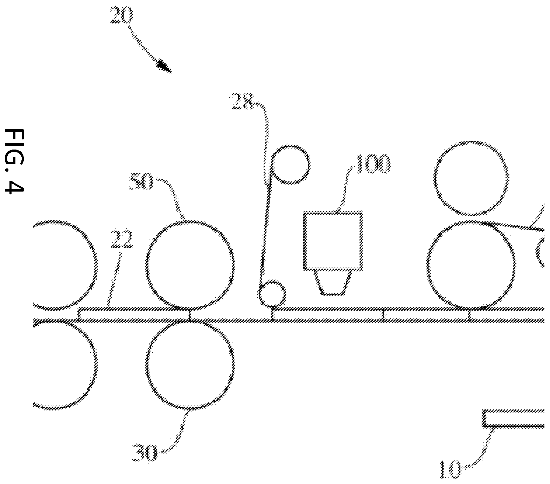

As shown in FIGS. 4-6, an embossed absorbent article of the invention and a method used to produce it can have a machine-direction 10 which extends longitudinally, and a lateral cross-direction 12 which extends transversely. The machine-direction 10 is the direction along which a particular component or material is transported along and through a particular position of a method for producing an embossed absorbent article. The cross-direction 12 lies generally within the plane of the material being transported through the method, and is aligned perpendicular to the machine-direction 10. Accordingly, in the view of the arrangement representatively shown in FIG. 4, the cross-direction 12 extends perpendicular to the plane of the sheet of the drawing.

An embossing method 20 for forming an embossed absorbent article can include positioning an absorbent 22 core on a web material, such as a topsheet 24. Moving the topsheet 24 and absorbent core 22, either of which may be in the form of a continuous web or individual components as shown in FIG. 4, along a machine-direction 10 at a selected web speed, and in certain embodiments the topsheet 24 and absorbent core 22 may pass through two or more precalendering rolls 21. Precalendering may be used as the embossing step can require very high forces to achieve a stable bond. By precalendering, some of this compression can be done prior to the actual embossing step. Precalendering is not intended to do any permanent deformation of the absorbent core 22, but rather to partially compress targeted regions of the absorbent core 22 prior to embossing. This allows the embossing step to "complete" the bond in the embossed regions; the remaining areas of the absorbent core 22 are allowed to "rebound" from the Precalendering step. Following the precalendering rolls 21 (if used) the topsheet 24 and absorbent core 22 are operatively contacted with a rotary embossing device 30 to form an embossment region 40 in the body facing surface 23 and a depression region 41 in the garment facing surface 27 of the absorbent core 22. An exemplary method for embossing an absorbent article is described in US 8,496,775 to Deng et al.

As shown in FIG. 4, the rotary embossing device 30 can be positioned cooperatively adjacent an anvil member 50. The anvil member 50 is oriented to counter-rotate relative to the rotary embossing device 30. Additionally, the anvil member 50 can be arranged to provide an operative embossing region which can be located between the rotary embossing device 30 and anvil member 50.

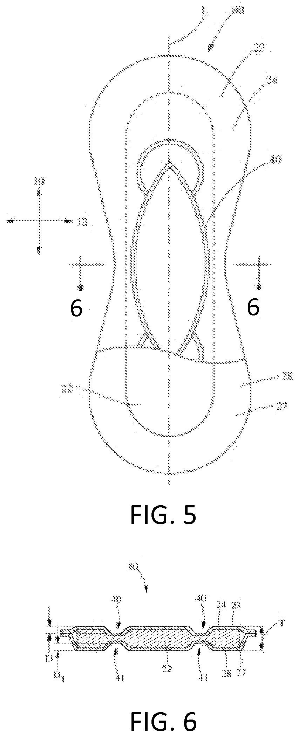

In certain embodiments, the absorbent core 22, if in web form, can be cut or otherwise divided to provide individual absorbent cores for use in feminine care articles, such as the feminine sanitary napkin shown in FIGS. 5 and 6. The feminine sanitary napkin 80 can have a lengthwise-dimension along the longitudinal direction 10, and a transverse-dimension along the laterally extending, cross-direction 12, which correspond respectively to the machine direction 10 and lateral cross-direction 12 described previously.

The absorbent core 22 is embossed before coming into contact with the backsheet 28. This allows the absorbent core 22 to be embossed to a greater depth in the embossed regions 40, while substantially avoiding undesired breaks or fractures of component portions of an absorbent article, such as a backsheet.

With reference to FIG. 4, the absorbent core 22 can be configured, for example by being positioned on a belt, to move at a selected speed in the machine-direction 10. In certain embodiments the speed can be from about 2 meters per second (m/s) to about 10 m/s, and in certain embodiments the speed can be from about 5 m/s to about 7 m/s.

FIGS. 5 and 6 show an embossed absorbent article of the present invention, which in this instance is a feminine sanitary napkin 80 having an absorbent core 22 positioned between a body facing surface 23 comprising a topsheet 24 and a garment facing surface 27 comprising a liquid impervious backsheet 28 joined to the topsheet 24, absorbent core 22, or both. The body facing surface 23 of the feminine sanitary napkin 80 has an embossment region 40 having a depth "D" as measured from the surrounding area of the body facing surface 23 to the lowermost portion of the embossment region 40. In certain embodiments the depth "D" of the embossment region 40 in the body facing surface 23 may be between about 20% to about 75% of the thickness "T" of the absorbent article, which in this instance is a feminine sanitary napkin, in certain other embodiments the depth "D" of the embossment region 40 may be between about 25% to about 50% of the thickness "T" of the absorbent article. The thickness of the absorbent article is measured at room temperature and at standard pressure and humidity. Embossment regions having this depth provide the benefit of improved body fit, good fluid barrier as well as aesthetic visual effects. The garment facing surface 27 of the feminine sanitary napkin 80 has a depression region 41 having a depth "D₁" as measured from the surrounding area of the body facing surface 27 to the lowermost portion of the depression region 41. In certain embodiments the depth " D₁" of the depression region 41 in the garment facing surface 27 may be between about 15% to about 75% of the thickness "T" of the absorbent article, which in this instance is a feminine sanitary napkin, in certain other embodiments the depth " D₁" of the depression region 41 may be between about 25% to about 50% of the thickness "T" of the absorbent article. While the garment facing surface 27 of the feminine sanitary napkin 80 is not directly contacted by the rotary embossing device 30, a depression region 41 is produced. While not being limited to theory it is thought that the depression region 41 in the garment facing surface 27 is formed from the tensioning of the topsheet, absorbent core and secondary topsheet (if present). Following embossment and the removal of the rotary embossing device and anvil member the recovery of the materials forming the topsheet, absorbent core, and secondary topsheet draws or pulls the materials back up towards the center of the embossment region and body facing surface of the feminine sanitary napkin.

The feminine sanitary napkin 80 has a longitudinal axis "L" and may also be provided with additional features commonly found in feminine sanitary napkins, such as "wings" or "flaps" as is known in the art or a fluid acquisition layer to promote fluid transport to the absorbent core 22. Further, the topsheet of the absorbent article can have various optional characteristics, as is known in the art, for example the topsheet can have apertures to aid in fluid acquisition.

In certain embodiments the topsheet may be compliant, soft feeling, and non-irritating to the wearers skin and hair. Further, the topsheet is liquid pervious, permitting liquids, such as menses or urine, to readily penetrate through its thickness. A suitable topsheet may be manufactured from a wide range of materials such as woven and nonwoven materials, for example a nonwoven web of fibers; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of: natural fibers, such as wood or cotton fibers; synthetic fibers, such as polymeric fibers -for example polyester, polypropylene, or polyethylene fibers; or from a combination of natural and synthetic fibers. When the topsheet comprises a nonwoven web, the web may be manufactured by a wide number of known techniques. For example, the web may be spunbonded, carded, wet-laid, melt-blown, hydroentangled, combinations of the above, or the like.

The backsheet is substantially impervious to liquids, such as menses or urine and may be manufactured from a thin plastic film, although other flexible liquid impervious materials may also be used. The backsheet prevents the exudates absorbed by the absorbent core from wetting a user's bedding or clothes, for example bedsheets, pants, pajamas and undergarments. In certain embodiments, the backsheet can operatively permit a sufficient passage of air and moisture vapor out of an absorbent article, particularly out of the absorbent core, while blocking the passage of bodily liquids. The backsheet may thus comprise: a woven or nonwoven material; polymeric films, such as thermoplastic films of polyethylene or polypropylene; or composite materials such as a film-coated nonwoven material. In one embodiment, the backsheet can be a breathable backsheet such as that described in U.S. Pat. No. 6,623,464 (Bewick-Sonntag et al.) issued 23 Sep 2003.

As shown in FIG. 6, the backsheet 28 and the topsheet 24 are positioned at the garment facing surface 27 and the body facing surface 23, respectively, of the feminine sanitary napkin 80. In certain embodiments the absorbent core can be joined with the topsheet, the backsheet, or both by known attachment means, such as those well known in the art. However, in certain embodiments of the present invention the absorbent core is unattached to the topsheet, the backsheet, or both.

The absorbent core 22 in FIGS. 5 and 6 is generally disposed between the topsheet 24 and the backsheet 28. The absorbent core 22 may comprise any absorbent material that is generally compressible, conformable, non-irritating to the wearer's skin, and capable of absorbing and retaining liquids such as urine and other certain body exudates, such as menses. The absorbent core 22 may comprise a wide variety of liquid-absorbent materials commonly used in feminine care articles and other absorbent articles, such as comminuted wood pulp, which is generally referred to as air felt. Examples of other suitable absorbent materials include creped cellulose wadding; melt blown polymers, including co-form; chemically stiffened, modified or crosslinked cellulosic fibers; tissue, including tissue wraps and tissue laminates; absorbent foams such as foams formed from High Internal Phase Emulsions (HIPEs); absorbent sponges; superabsorbent polymers; absorbent gelling materials; or any other known absorbent material or combinations of materials. The absorbent core may further comprise minor amounts (typically less than 10%) of non-liquid absorbent materials, such as adhesives, waxes, oils and the like. Examples of absorbent structures that may be used in the present invention are found in U.S. Pat. No. 4,834,735 (Alemany et al.) issued 30 May 1989; U.S. Pat. No. 5,625,222 (DesMarais et al.) 22 Jul 1997.

The absorbent core may also include one or more superabsorbent materials. Superabsorbent materials suitable for use in the present invention are known to those skilled in the art, and may be in any operative form, such as particulate form. The superabsorbent material can be a water-swellable, generally water-insoluble, hydrogel-forming polymeric absorbent material, which is capable of absorbing at least about 20, in certain embodiments about 30, and in additional embodiments about 60 times or more its weight in physiological saline (for example 0.9 wt % NaCl). The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers are preferably lightly crosslinked to render the material substantially water insoluble. Crosslinking may, for example, be by irradiation or covalent, ionic, Van der Waals, or hydrogen bonding. Suitable materials are available from various commercial vendors, such as the Dow Chemical Company and Stockhausen, Inc. The superabsorbent material may be included in an appointed storage or retention portion of the absorbent article, and may optionally be employed in other components or portions of the absorbent article.

Referring back to FIG. 1 a method for producing an embossed absorbent article of the present invention can include attaching a backsheet 28 to the topsheet 24, absorbent core 22 or both. In certain embodiments the backsheet 28 may be attached to the topsheet 24, absorbent core 22, or both after contacting the absorbent core 22 with the rotary embossing device 30. As shown in FIG. 6, portions of the backsheet 28 will conform to the contours of the depression regions 41, thereby creating an irregular surface. The various portions or components of each absorbent article, such as the absorbent core 22, topsheet 24 or backsheet 28 can be joined or secured together employing any operative technique. A variety of suitable mechanisms known to one of skill in the art may be utilized to achieve any such secured relation. Examples of such securing mechanisms or systems can include, but are not limited to, the application of adhesives in a variety of patterns between the two adjoining surfaces, entangling at least some portions of one absorbent body component with portions of the adjacent surface of another component, or fusing at least portions of the adjacent surface of one component to portions of another component of the absorbent.

Although the apparatuses and methods are described in the context of the feminine hygiene article shown in FIGS. 5 and 6, it is to be appreciated that the methods and apparatuses herein may be used to assemble and emboss various substrates and/or elastic laminates that can be used with various process configurations and/or absorbent articles, such as for example, incontinence pads, diapers, or diaper pants.

### Embossing Apparatus

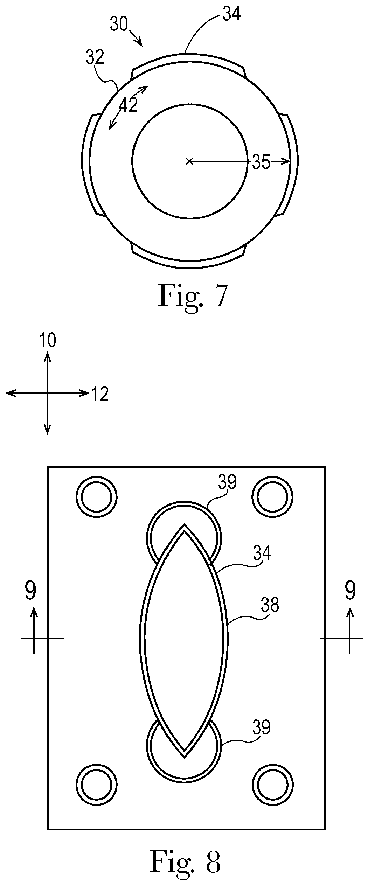

The embossing apparatuses includes an embossing roll and an anvil roll. The embossing roll includes a plurality of embossing elements, or nubs, protruding radially outward, wherein each nub includes an embossing surface. The embossing roll is adjacent the anvil roll to define a nip between the embossing surfaces and the anvil roll. The rotary embossing device 30, as shown in FIG. 7, includes an outer peripheral surface 32 having a lateral cross-direction 12 and a circumferential-direction 42, and an embossing member 34 located on the outer surface 32. The rotary embossing device 30 can have a selected roll radius 35. In certain embodiments the roll radius can be from about 7 cm to about 25 cm. In certain other embodiments the roll radius can be from about 11 cm to about 19 cm.

Any conventional power mechanism or system can be employed to drive the rotary embossing device 30. Such power mechanisms can include engines, motors, electro-magnetic power systems, fluidic power systems, or the like, as well as combinations thereof. The selected drive system can be configured to provide the rotary embossing device 30 with a selected surface speed at the outer peripheral rim surface 32, and certain embodiments, the peripheral surface speed can be configured to substantially equal the web speed of the absorbent core that is to be embossed.

The rotary embossing device 30 can have an outer peripheral rim surface 32 which extends along the circumferential direction 42 and along the transverse cross-direction 12 of the rotary embossing device. With reference to FIGS. 4, 7, 8, and 9, at least one embossing member 34 can be located on the outer peripheral surface 32 of the rotary embossing device 30. In certain embodiments a plurality of two or more embossing members 34 can be distributed over the outer peripheral surface 32 in a desired array. For example, the plurality of embossing members can be arranged in series along the circumferential direction of the embossing device 30, and the serial arrangement may be irregular or substantially regular, as desired.

While the embossing member 34 can be of any desired shape, design or combination of shapes and designs for providing the desired embossment to an absorbent article an embossing member 34 in certain embodiments, as shown in FIG. 8 may comprise a primary shape 38 bordered by two or more secondary shapes 39.

An embossing member may provide to an absorbent article a symmetrical shape, an asymmetrical shape, a regular or irregular rectilinear shape, a regular or irregular curvilinear shape or the like, as well as combinations thereof. The embossing member may be configured to be discontinuous or substantially continuous, as desired. In particular arrangements, the embossing member can be arranged to effectively provide a substantially closed-shape. In certain embodiments the embossing member can be configured to extend along substantially an entirety of the absorbent core perimeter during the embossing operation.

### Embossing Nubs

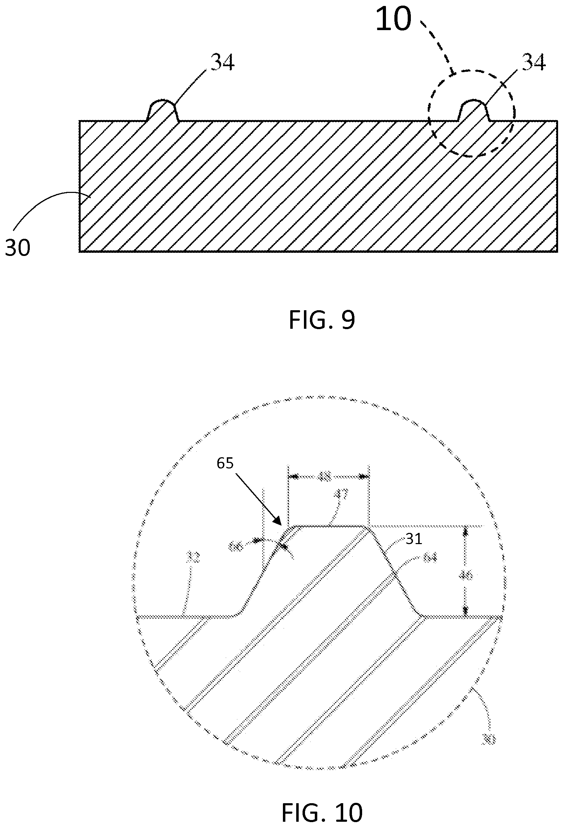

An embossing member comprises one or more embossing nubs. As shown in FIG. 9, a cross-sectional view of FIG. 8, embossing device 30 comprises embossing members 34. FIG. 10 shows an enlarged embossing nub 31. Embossing nub 31 is and has a nub height 46 and a nub width 48. The nub height 46 is the distance between the topmost portion of the nub embossing surface 47 and a corresponding local outer-surface region 32 of the embossing device 30. In certain embodiments, the nub height 46 can be from about 2mm to about 12mm, or from about 3mm to about 4mm. A plurality of nubs may be configured with the same or different heights. In certain embodiments the nub width 48, or embossing surface width, can be from about 0.25mm to about 4mm, or from about 0.5mm to about 1.5mm. The embossing nub 31 can include circumferential sidewall 64 comprising a sidewall angle 66. Sidewall 64 is substantially tapered. In certain embodiments the sidewall angle 66 can be from about 0 degrees to about 50 degrees, or from about 10-20 degrees.

The embossing nub 31 comprises a shoulder (or edge break) 65 located between the sidewall 64 and the embossing surface 47. Shoulder 65 is radiused (curvilinear). The radius R is from 0.1mm to 1mm, from 0.3mm to 0.5mm, or about 0.35mm. Nubs with significant shoulders, or edge breaks, have been proven to improve emboss and reduce the tendency of a substrate, e.g., a topsheet, to peel. The radiused nubs minimize the applied process strain of the topsheet so that the applied process strain is less than the failure strain of the topsheet of interest. This way, the topsheet won't be cut by the nub and the topsheet will not comprise the topsheet pop-out defects shown, *e.g.,* in FIGS. 1 and 2. The applied strain is a function of the dimensions described in this section.

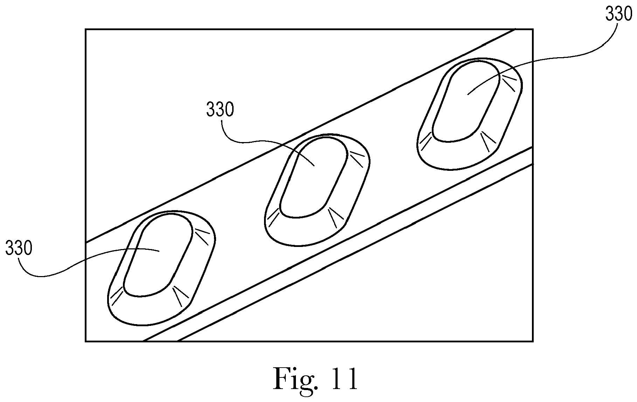

The circumferential sidewall 64 defines an outer perimeter of the nub 31. The nubs may have a perimeter that defines circular, square, rectangular, elliptical, obround (see, *e.g.,* the three embossing nubs 330 in FIG. 11), parallelogram, tear drop, and various types of other shapes; one embossing member or embossing device may comprise a combination of two or more different nub shapes. For example, the nubs may have a perimeter that defines an elliptical shape. In some instances, the nubs may be configured such that resulting emboss regions also offer aesthetic benefits such as, for example, a stitched-like appearance along with a relatively smooth texture feel to the skin. Various quantities of nubs may be arranged in groupings, or patterns, to form discrete emboss sites (rather than long channels, for example, which are often found with embossing). Spacing between nubs may be 0.5mm to 5mm.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. An apparatus for embossing substrates, the apparatus comprising:
a. an anvil member (50); and
b. an embossing device (30)comprising a base surface and plurality of embossing members (34) extending from the base surface, each of the plurality of embossing members comprising:
i. a sidewall (64);
ii. an embossing surface (47) having a width (48); and
iii. a shoulder (65) connecting the sidewall to the embossing surface;
wherein the shoulder comprises a radius of from 0.1mm to 1mm; wherein the embossing surface width is from 0.25mm to 4mm; and wherein the sidewall is substantially tapered from the base surface.

2. The apparatus of claim 1, wherein the radius is from 0.3mm to 0.5mm.

3. The apparatus of claims 1 and 2, wherein the radius is 0.35mm.

4. The apparatus of any of the preceding claims, wherein the embossing surface width is from 0.5mm to 1.5mm.

5. The apparatus of any of the preceding claims, wherein the sidewall has an angle of from 0 to 50 degrees.

6. The apparatus of any of the preceding claims, wherein each nub comprises a height of from 2mm to 12mm.

7. A method for embossing substrates, the method comprising the steps of:
a. providing an anvil member (50);
b. providing an embossing device (30) comprising a base surface and plurality of embossing members (34) extending from the base surface, each of the plurality of embossing members comprising:
i. a sidewall (64);
ii. an embossing surface (47) having a width (48); and
iii. a shoulder (65) connecting the sidewall to the embossing surface;
wherein the shoulder is comprises a radius of from 0.1mm to 1mm;
wherein the embossing surface width is from 0.25mm to 4mm; and wherein the sidewall is substantially tapered from the base surface; and
c. advancing a topsheet an absorbent core between the anvil member and the embossing device so that emboss regions are formed via the plurality of embossing members.

8. The method of claim 7, wherein the radius is from 0.3mm to 0.5mm or preferably 0.35 mm.

9. The method of claim 7, wherein the embossing surface width is from 0.5mm to 1.5mm and the sidewall has an angle of from 0 to 50 degrees.

10. The method of claim 7, wherein each embossing member comprises a height of from about 2mm to about 12mm.

## Patentansprüche

1. Vorrichtung zum Prägen von Substraten, wobei die Vorrichtung Folgendes umfasst:
a. ein Ambosselement (50); und
b. eine Prägeanordnung (30) umfassend eine Basisfläche und eine Vielzahl von Prägeelementen (34), die von der Basisfläche ausgehen, wobei jedes der Vielzahl von Prägeelementen umfasst:
i. eine Seitenwand (64);
ii. eine Prägefläche (47) mit einer Breite (48); und
iii. eine Schulter (65), die die Seitenwand mit der Prägefläche verbindet; wobei die Schulter einen Radius zwischen 0,1 mm und 1 mm umfasst;
wobei die Breite der Prägefläche von 0,25 mm bis 4 mm beträgt; und wobei sich die Seitenwand im Wesentlichen von der Basisfläche ausgehend verjüngt.

2. Vorrichtung nach Anspruch 1, wobei der Radius von 0,3 mm bis 0,5 mm beträgt.

3. Vorrichtung nach Anspruch 1 und 2, wobei der Radius 0,35 mm beträgt.

4. Vorrichtung nach einem der vorhergehenden Ansprüche, wobei die Breite der Prägefläche von 0,5 mm bis 1,5 mm beträgt.

5. Vorrichtung nach einem der vorhergehenden Ansprüche, wobei die Seitenwand einen Winkel von 0 bis 50 Grad aufweist.

6. Vorrichtung nach einem der vorhergehenden Ansprüche, wobei jede Noppe eine Höhe zwischen 2 mm und 12 mm umfasst.

7. Verfahren zum Prägen von Substraten, wobei das Verfahren die folgenden Schritte umfasst:
a. Bereitstellen einer Ambossrolle (50);
b. Bereitstellen einer Prägeanordnung (30) umfassend eine Basisfläche und eine Vielzahl von Prägeelementen (34), die von der Basisfläche ausgehen, wobei jedes der Vielzahl von Prägeelementen umfasst:
i. eine Seitenwand (64);
ii. eine Prägefläche (47) mit einer Breite (48); und
iii. eine Schulter (65), die die Seitenwand mit der Prägefläche verbindet;
wobei die Schulter einen Radius von 0,1 mm bis 1 mm umfasst;
wobei die Breite der Prägefläche von 0,25 mm bis 4 mm beträgt und
wobei sich die Seitenwand im Wesentlichen von der Basisfläche ausgehend verjüngt; und
c. Vorschieben einer Oberschicht und eines Absorptionskerns zwischen dem Ambosselement und der Prägeanordnung derart, dass über die Vielzahl von Prägeelementen Prägebereiche gebildet werden.

8. Verfahren nach Anspruch 7, wobei der Radius von 0,3 mm bis 0,5 mm oder vorzugsweise 0,35 mm beträgt.

9. Verfahren nach Anspruch 7, wobei die Breite der Prägefläche von 0,5 mm bis 1,5 mm beträgt und die Seitenwand einen Winkel von 0 bis 50 Grad aufweist.

10. Verfahren nach Anspruch 7, wobei jedes Prägeelement eine Höhe von etwa 2 mm bis etwa 12 mm umfasst.

## Revendications

1. Appareil pour le gaufrage de substrats, l'appareil comprenant :
a. un élément enclume (50) ; et
b. un dispositif de gaufrage (30) comprenant une surface de base et une pluralité d'éléments de gaufrage (34) s'étendant depuis la surface de base, chaque élément de la pluralité d'éléments de gaufrage comprenant :
i. une paroi latérale (64) ;
ii. une surface de gaufrage (47) ayant une largeur (48) ; et
iii. un épaulement (65) reliant la paroi latérale à la surface de gaufrage ;
dans lequel l'épaulement comprend un rayon allant de 0,1 mm à 1 mm ; dans lequel la largeur de la surface de gaufrage va de 0,25 mm à 4 mm ; et dans lequel la paroi latérale est essentiellement conique depuis la surface de base.

2. Appareil selon la revendication 1, dans lequel le rayon va de 0,3 mm à 0,5 mm.

3. Appareil selon la revendication 1 et 2, dans lequel le rayon est de 0,35 mm.

4. Appareil selon l'une quelconque des revendications précédentes, dans lequel la largeur de la surface de gaufrage va de 0,5 mm à 1,5 mm.

5. Appareil selon l'une quelconque des revendications précédentes, dans lequel la paroi latérale a un angle allant de 0 à 50 degrés.

6. Appareil selon l'une quelconque des revendications précédentes, dans lequel chaque point central présente une hauteur allant de 2 mm à 12 mm.

7. Procédé de gaufrage de substrats, le procédé comprenant les étapes consistant à :
a. fournir un rouleau enclume (50) ;
b. fournir un dispositif de gaufrage (30) comprenant une surface de base et une pluralité d'éléments de gaufrage (34) s'étendant depuis la surface de base, chaque élément de la pluralité d'éléments de gaufrage comprenant :
i. une paroi latérale (64) ;
ii. une surface de gaufrage (47) ayant une largeur (48) ; et
iii. un épaulement (65) reliant la paroi latérale à la surface de gaufrage ;
dans lequel l'épaulement comprend un rayon allant de 0,1 mm à 1 mm ;
dans lequel la largeur de la surface de gaufrage va de 0,25 mm à 4 mm ; et
dans lequel la paroi latérale est essentiellement conique depuis la surface de base ; et
c. faire avancer une feuille de dessus d'une âme absorbante entre l'élément enclume et le dispositif de gaufrage, de sorte que des régions de gaufrage soient formées par le biais de la pluralité d'éléments de gaufrage.

8. Procédé selon la revendication 7, dans lequel le rayon va de 0,3 mm à 0,5 mm ou vaut de préférence 0,35 mm.

9. Procédé selon la revendication 7, dans lequel la largeur de la surface de gaufrage va de 0,5 mm à 1,5 mm et la paroi latérale présente un angle allant de 0 à 50 degrés.

10. Procédé selon la revendication 7, dans lequel chaque élément de gaufrage présente une hauteur allant d'environ 2 mm à environ 12 mm.
